# EUROPEAN PATENT APPLICATION

(11) **EP 4 596 671 A1**
(43) Date of publication of application: **06.08.2025**
(21) Application number: 23872652.5
(22) Date of filing: 02.10.2023
(51) Int. Cl.: C12M 3/00, C12N 5/071

(54) **LUMINAL STRUCTURE, AND METHOD FOR MANUFACTURING LUMINAL STRUCTURE**

(30) Priority: 30.09.2022 JP 2022158455
(71) Applicant: Tohoku University, Sendai-shi, Miyagi 980-8577 (JP)
(72) Inventor: ONOE, Hiroaki, Yokohama-shi Kanagawa 223-8522 (JP); TANAKA, Shuma, Yokohama-shi Kanagawa 223-8522 (JP); ITAI, Shun, Yokohama-shi Kanagawa 223-8522 (JP); TOYOHARA Takafumi, Sendai-shi Miyagi 980-8577 (JP)
(74) Representative: van Trier, Norbertus Henricus Gerardus
(86) International application number: PCT/JP2023/035893
(87) International publication number: WO 2024/071433

(57) **Abstract**

[Problem] To provide the method for manufacturing the luminal structure, which can three-dimensionally adjust the asperities in the luminal structure by generating the bubbles with a rod-shaped mold for forming a body cavity of the luminal structure.

[Solution] A method for manufacturing a luminal structure, comprising:an in-cast temporary fixative obtaining step of obtaining an in-cast temporary fixative as a temporary fixative housed in a cast having a recess corresponding to an outer shape of the luminal structure, a core forming member corresponding to a lumen body of the luminal structure being inserted in the temporary fixative; and an in-cast temporary fixative solidifying step of solidifying the in-cast temporary fixative in a state of a bubble being generated in the in-cast temporary fixative using the core forming member.

## Description

### TECHNICAL FIELD

This invention relates to a luminal structure such as an artificial intestine and a method for manufacturing the luminal structure. More specifically, this invention relates to a method for manufacturing a luminal structure, such as a method which can adjust three-dimensional asperities by air bubbles.

### BACKGROUND ART

Japanese Unexamined Patent Application Publication (Translation of PCT Application) No. 2020-500018 describes an artificial intestine tissue. There has been known a technique of artificially manufacturing an intestine as in this literature.

For this invention, there has been demanded a technique of manufacturing a luminal structure, such as an artificial intestine, having a proper structure by three-dimensionally adjusting asperities in the luminal structure.

### CITATION LIST

### PATENT LITERATURE

Patent Literature 1: Japanese Unexamined Patent Application Publication (Translation of PCT Application) No. 2020-500018

### SUMMARY OF INVENTION

### TECHNICAL PROBLEM

This invention provides a method for manufacturing a luminal structure, which can three-dimensionally adjust asperities in the luminal structure, and also provides such a luminal structure.

### SOLUTION TO PROBLEM

This invention is based on findings from an example where asperities in a luminal structure can be three-dimensionally adjusted using bubbles.

The method for manufacturing the luminal structure according to this invention includes an in-cast temporary fixative obtaining step and an in-cast temporary fixative solidifying step. Examples of the luminal structure include an artificial intestine.

The in-cast temporary fixative obtaining step is a step of obtaining an in-cast temporary fixative. The in-cast temporary fixative is a temporary fixative housed in a cast having a recess corresponding to the outer shape of the luminal structure, and a core forming member corresponding to a lumen body of the luminal structure is inserted in the temporary fixative. Examples of the temporary fixative include a pregel solution.

The in-cast temporary fixative solidifying step is a step of solidifying the in-cast temporary fixative in a state of bubbles being generated using the core forming member in the in-cast temporary fixative. The core forming member may function as an electrode. This step may further include an electrolyzation step of generating the bubbles on the surface of the core forming member by electrolyzation using the core forming member as the electrode. The value of voltage applied to the core forming member and the time of such voltage application are adjusted so that the shape of asperities inside the resultant luminal structure can be adjusted.

After this step, a step of taking the solidified in-cast temporary fixative out of a cast, a step of washing the in-cast temporary fixative taken out of the cast, and the like may be performed to obtain the luminal structure.

The luminal structure of this invention may be obtained by the above-described manufacturing method. The luminal structure of this invention is a luminal structure having a tubular shape, which includes a lumen. The lumen includes a plurality of recesses having a depth of 10 µm or more and 200 µm or less and a maximum width of 20 µm or more and 500 µm or less. The luminal structure of this invention preferably has 10² recesses or more.

### ADVANTAGEOUS EFFECTS OF INVENTION

This invention can provide the method for manufacturing the luminal structure, which can three-dimensionally adjust the asperities in the luminal structure by generating the bubbles with a rod-shaped mold for forming a body cavity of the luminal structure.

### BRIEF DESCRIPTION OF DRAWINGS

Fig. 1 is a conceptual view for describing a luminal structure.
Fig. 2 is a drawing showing an example of a manufacturing system in an example. Fig. 2(a) is a conceptual view of a cast and a rod-shaped mold used in the example. Fig. 2(b) is a conceptual view of the system used in the example.
Fig. 3 is a conceptual view showing the state of the progress of electrolyzation.
Fig. 4 is a photograph as a view showing the state of the progress of electrolyzation.
Fig. 5 is a photograph as a view showing an artificial intestine obtained by the example.
Fig. 6 is a photograph as a view showing an experimental system used for a cell cultivation experiment.
Fig. 7 is a photograph as a view showing a Caco-2 cell in the artificial intestine.
Fig. 8 is a graph as a view showing the diameter of a tube of the artificial intestine, the depth of a recess formed in a lumen, and the diameter of the opening of the recess.
Fig. 9 is a photograph as a view showing a cocultivation system.
Fig. 10 is a fluorescence photograph as a view showing the results of cocultivation.
Fig. 11 is an image captured by a phase-contrast microscope as a view showing the results of observation..

### DESCRIPTION OF EMBODIMENTS

Hereinafter, embodiments of the present invention will be described with reference to the drawings. The present invention is not limited to the embodiments described below, and also includes modifications made as necessary within a scope obvious to those skilled in the art from the embodiments below.

A method for manufacturing a luminal structure according to this invention includes an in-cast temporary fixative obtaining step and an in-cast temporary fixative solidifying step.

### Luminal Structure

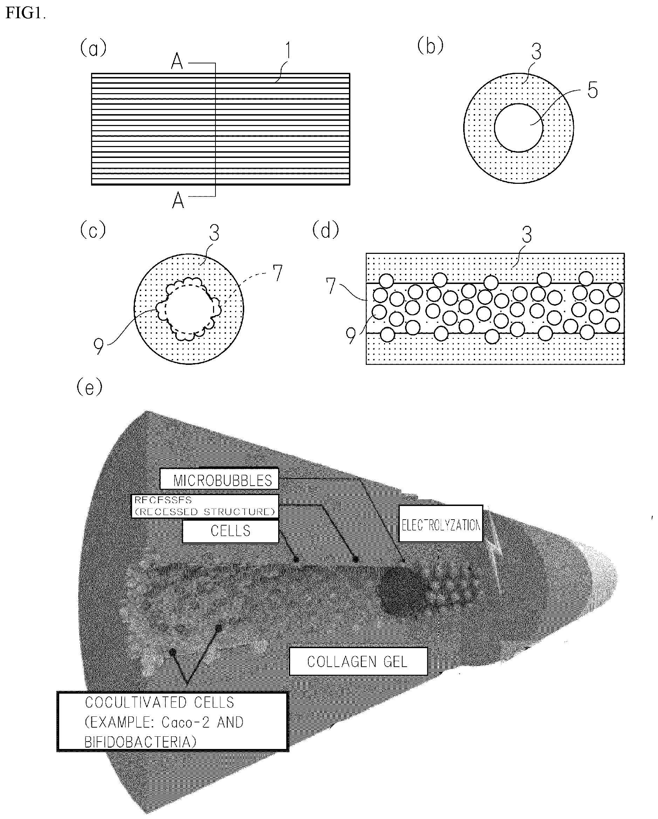

Fig. 1 is a conceptual view for describing the luminal structure. Fig. 1(a) shows an example of a side view of the luminal structure, Fig. 1(b) shows an example of a front view (end portion) of the luminal structure, Fig. 1(c) shows an example of a sectional view taken along A-A line of Fig. 1(a), and Fig. 1(d) shows an example of a sectional view of Fig. 1(a). Fig. 1(e) is a conceptual view for describing the luminal structure and examples. As shown in Fig. 1, the luminal structure 1 has a body 3 of the luminal structure and a lumen 5 formed inside the body 3. The outer shape of the luminal structure is a so-called tubular shape. The lumen 5 has a cylindrical lumen body 7 and a plurality of recesses 9 formed in the region of the body 3 of the luminal structure. In the example shown in Fig. 1(b), the lumen 5 has, in the region of the end portion of the luminal structure, no recesses 9, but has only the lumen body 7. However, in the luminal structure of this invention, the lumen 5 may also have the recesses 9 in the region of the end portion. The luminal structure may be flexible.

The luminal structure preferably has a size and physical properties similar to those of part of the intestines of a human or a mammal. The luminal structure is in a tubular shape, for example. The length of the luminal structure may be adjusted as necessary according to use application. Moreover, the thickness of a wall forming the body 3 of the luminal structure may also be adjusted as necessary according to use application. When the length of the tube is lₜ, the length may be 1 mm ≤ lₜ ≤ 1 m, 1 mm ≤ lₜ ≤ 10 cm, 1 mm ≤ lₜ ≤ 1 cm, 5 mm ≤ lₜ ≤ 1 m, 5 mm ≤ lₜ ≤ 50 cm, or 5 mm ≤ lₜ ≤ 10 cm. When the average radius of the outer periphery of the tube is rt, the average radius may be 0.1 mm ≤ rₜ ≤ 3 cm, 0.6 mm ≤ rₜ ≤ 1 cm, 0.6 mm ≤ rₜ ≤ 5 mm, or 0.6 mm ≤ rₜ ≤ 3 mm. When the average diameter of the lumen (lumen body 7) of the tube is dₜ, the average diameter may be 0.1rₜ ≤ dₜ ≤ 1.8rₜ, 0.1rt ≤ dₜ ≤ 0.8rₜ, 0.2rt ≤ dₜ ≤ 0.8rₜ, 0.2rₜ ≤ dₜ ≤ 0.6rₜ, or 0.3rₜ ≤ dₜ ≤ 0.6rₜ. For the luminal structure, a well-known resin having biocompatibility may be used as necessary. An artificial intestine is well-known, and therefore, the luminal structure can be manufactured using a well-known material.

The recesses 9 are asperities formed intentionally, and a recess may be further formed in the recess. Examples of the recess 9 include a substantially hemispherical recess, and such a recess has a depth of no less than 10um and no more than 200um and a width of no less than 20µm and no more than 500um. The luminal structure is only required to have the plurality of recesses 9 within these ranges. The depth and width of the formed recess 9 can be considered as equal to the radius and diameter of a bubble. Thus, the depth of the recess 9 is, for example, no less than 10um and no more than 200um, and may be no less than 20 µm and no more than 150 µm, no less than 20 µm and no more than 100 µm, no less than 50 µm and no more than 150 µm, or no less than 50 µm and no more than 100 µm. The width of the recess 9 is, for example, no lees than one time and no more than four times or less the above-described depth of the recess 9, and may be no less than 1.2 times and no more than 3 times or no less than 1.3 times and no more than 2.5 times. The luminal structure of this invention preferably has recesses 9 of 1 × 10² or more which is equivalent to any of the above-described ranges. The number of recesses 9 may be no less than 1 × 10² and no more than 1 × 10²⁰ , no less than 2 × 10² and no more than 1 × 10¹⁰, no less than 1 × 10³ and no more than 1 × 10⁹, or no less than 1 × 10³ and no more than 1 × 10⁸.

Examples of the luminal structure include an artificial intestine. Examples of the intestine include a small intestine and a large intestine. Examples of the small intestine include the duodenums, the jejuna, and the ilea. Examples of the large intestine include the ceca, the colons, and the recta. The colons include the ascending colon, the transverse colon, the descending colon, and the sigmoid colon. The artificial intestine may be an artificial organ partially or entirely replaced with any of these parts. Thus, the size and thickness of the artificial intestine may be adjusted as necessary according to use application. The intestine is preferably an artificial intestine for a human. That is, preferable examples of the artificial intestine include a medical device for treatment of a human.

### In-cast Temporary Fixative Obtaining Step

The in-cast temporary fixative obtaining step is a step for obtaining an in-cast temporary fixative. The in-cast temporary fixative is a temporary fixative housed in a cast having recesses corresponding to the outer shape of the luminal structure and having therein a core forming member corresponding to the lumen body of the luminal structure. The temporary fixative means an agent before cured by a subsequent process. The temporary fixative may be in a liquid form or a semisolid state. Examples of the temporary fixative include a thermosetting resin, a light curing resin, an adhesive, an impression material, and a pregel solution. The thermosetting resin is a resin solidified by heat application. The light curing resin is a resin solidified by light irradiation. The adhesive is a resin solidified by moisture evaporation. The pregel solution is a semisolid liquid containing a predetermined resin component and turning into a gel by heat application or air drying as described in, for example, Japanese Patent No. 7129668, Japanese Unexamined Patent Application Publication No. 2020-180239, and Japanese Unexamined Patent Application Publication (Translation of PCT Application) No. 2022-513229. In this step, a circular columnar structure corresponding to the outer shape of the luminal structure may be manufactured, and thereafter, a core portion may be removed and the core forming member may be inserted therein. In this step, a circular columnar structure corresponding to the outer shape of the luminal structure may be manufactured, and thereafter, the core forming member may be inserted and a core portion may be removed. In this step, the core forming member may be inserted into the cast in advance, and the in-cast temporary fixative may be obtained with a core portion (corresponding to the lumen body 7) removed.

### In-cast Temporary Fixative Solidifying Step

The in-cast temporary fixative solidifying step is a step of solidifying the in-cast temporary fixative in a state of bubbles being generated in the in-cast temporary fixative using the core forming member. These bubbles are intentionally or artificially generated. Moreover, the amount of bubbles generated and the positions of the bubbles generated are preferably controlled. The core forming member may function as an electrode. This step may further include an electrolyzation step of generating air bubbles on the surface of the core forming member by electrolyzation using the core forming member as the electrode. The value of voltage applied to the core forming member and the time of such voltage application are adjusted so that the shape of the asperities inside the resultant luminal structure can be adjusted. The core forming member may have, in the surface thereof, a plurality of micro holes (having such a size that the in-cast temporary fixative is less likely to enter the core forming member), and may form bubbles by releasing fluid (for example, air or oil) from the inside of the core forming member. For example, in a case where the fluid is the oil, portions where the oil is present are not solidified and form the recesses 9. After this step, a step of taking the solidified in-cast temporary fixative out of the cast, a step of washing the in-cast temporary fixative taken out of the cast, and the like may be performed to obtain the luminal structure.

The luminal structure of this invention may be obtained by the above-described manufacturing method. The artificial intestine can be used for various medical practices and medical-related experiments. Hereinafter, the artificial intestine will be described as an example of this invention. However, this invention is not limited to the following examples, and also includes those for which the well-known techniques are employed as necessary.

### Examples

### [Experimental Example 1]

### Manufacturing of Artificial Intestine

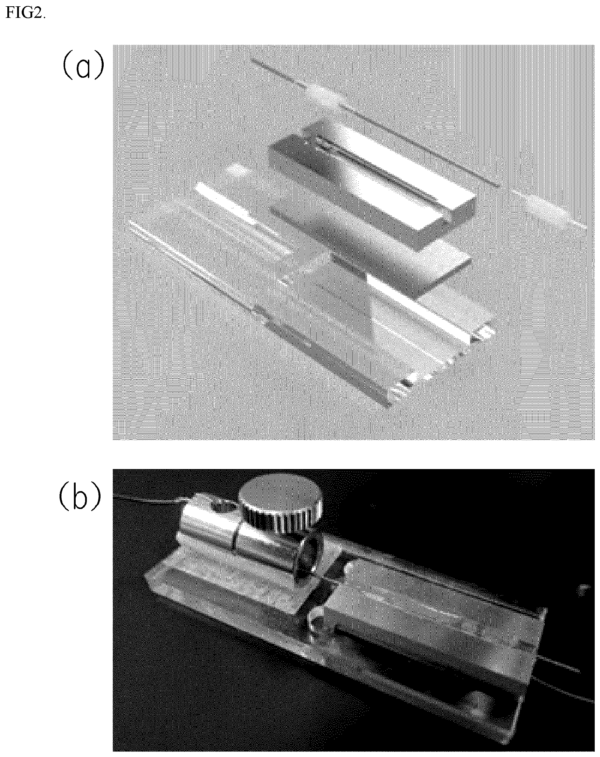

For creating an artificial intestine, the following cast and rod-shaped mold were prepared. Fig. 2 is a drawing showing an example of a manufacturing system in the example. Fig. 2(a) is a conceptual view of the cast and the rod-shaped mold used in the example. Fig. 2(b) is a conceptual view of the system used in the example. The rod-shaped mold corresponds to a hollow core portion. A cast obtained by cutting a collagen pregel solution injection groove in an aluminum piece of 10 × 25 × 3 was used. The cast is not limited to aluminum as long as the material thereof has conductivity. A tungsten rod-shaped mold having a diameter of 0.5 mm was used. The rod-shaped mold is not limited to tungsten as long as the material thereof has conductivity.

The cast was filled with a collagen pregel solution (AteloCell^{®}). Using the rod-shaped mold as an electrode, a DC voltage of 5 V was applied for 200 msecs through the tungsten wire, and in this manner, air bubbles were generated by electrolyzation of the pregel. Under 37°C, the resultant was left stand for two hours, and in this manner, the gel was cured. A collagen tube obtained as a result of curing of the gel was taken out of the cast, and in this manner, the artificial intestine was obtained.

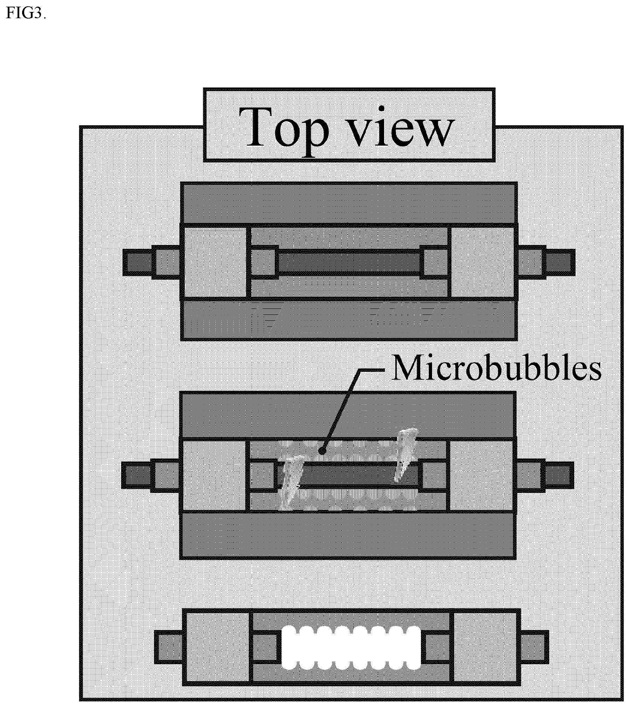

Fig. 3 is a conceptual view showing the state of the progress of electrolyzation. The upper view shows the state before electrolyzation. The middle view shows the state when electrolyzation starts and microbubbles (fine bubbles) are generated. The lower view shows the state when electrolyzation progresses and many microbubbles are generated around the tungsten wire in the collagen pregel solution.

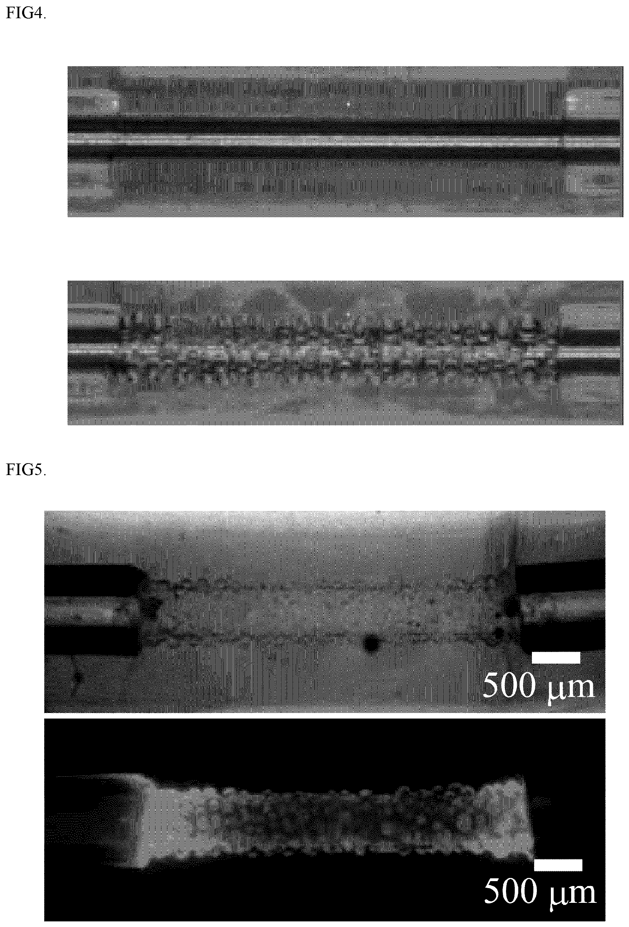

Fig. 4 is a photograph as a view showing the state of the progress of electrolyzation. The upper image shows the state before electrolyzation, and the lower image shows the state when electrolyzation progresses and many microbubbles are generated around the tungsten wire in the collagen pregel solution.

Fig. 5 is a photograph as a view showing the artificial intestine obtained by the example. The upper view shows a phase-contrast image, and the lower view shows a fluorescence micrograph.

The outer and inner diameter dimensions (average ± standard deviation) of the artificial intestine obtained using the cast having the groove with a radius of 1.2 mm and the rod-shaped mold having a diameter of 0.5 mm were 2454 ± 24 µm and 538 ± 22 µm.

By changing the dimensions of the cast and the rod-shaped mold, the artificial intestine with a desired size can be obtained. An extremely large outer diameter causes degradation of medium permeability, and an extremely large inner diameter causes an increase in the number of days of cultivation required until a cell becomes confluent. For these reasons, the inner diameter is preferably 500 µm or less.

The shape of the recess can be controlled by performing treatment on the surface of the rod-shaped mold. For example, in a case where a water-repelling coating (OPTOOL HD-1100TH, DAIKIN, Japan) was applied to the surface, the contact angle of the air bubble can be changed. A decrease in the contact angle results in expansion of the opening of the recess, which is advantageous because subsequent cell seeding is facilitated.

The size of the recess can be controlled by the time of voltage application in electrolyzation. In the case of using a voltage of 5 V, when the application time increases to 25 msecs, 100 msecs, 200 msecs, and 1000 msecs, the diameter (average ± standard deviation) of the air bubbles generated increases to 64.5 ± 22.3 µm, 76.5 ± 26.2 µm, 116.6 ± 28.7 µm, and 131.2 ± 33.8 µm. When the voltage application time exceeds 200 msecs, the generated air bubbles are joined to air bubbles therearound to form a larger air bubble, and as a result, the number of recesses decreases.

In a case where the cell is cultivated using a collagen gel (4.0 mg/mL), the depth is preferably 100 µm or more in consideration of deformation of the recess due to the traction force of the cell. When the depth is less than such a value, the recess is flattened in some cases.

The hardness of the tube can be further increased by increasing the concentration of the collagen gel or using gelatin. In this case, a depth of less than 100 µm causes no problem.

### [Experimental Example 2]

### Cultivation of Human Cell

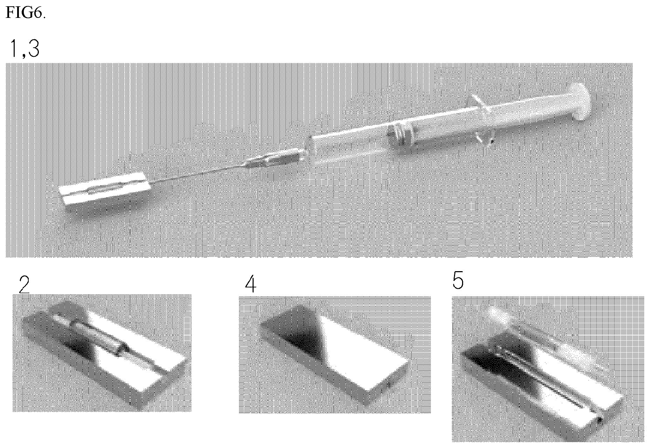

Fig. 6 is a view showing an experimental system used for a cell cultivation experiment. As shown in 1 of Fig. 6, a Caco-2 cell suspension (1.0 × 10⁷ cells/mL) purchased from the Institute of Physical and Chemical Research was injected into a syringe (250 µL, Hamilton), and was injected into the collagen gel lumen obtained after removal of the rod-shaped mold. As shown in 2 of Fig. 6, the resultant was incubated at 37°C in 5% CO₂. As shown in 3 of Fig. 6, the human cell (Caco-2 cell) was removed again. As shown in 4 of Fig. 6, the resultant was incubated with placed upside down. The artificial intestine was taken out of the cast.

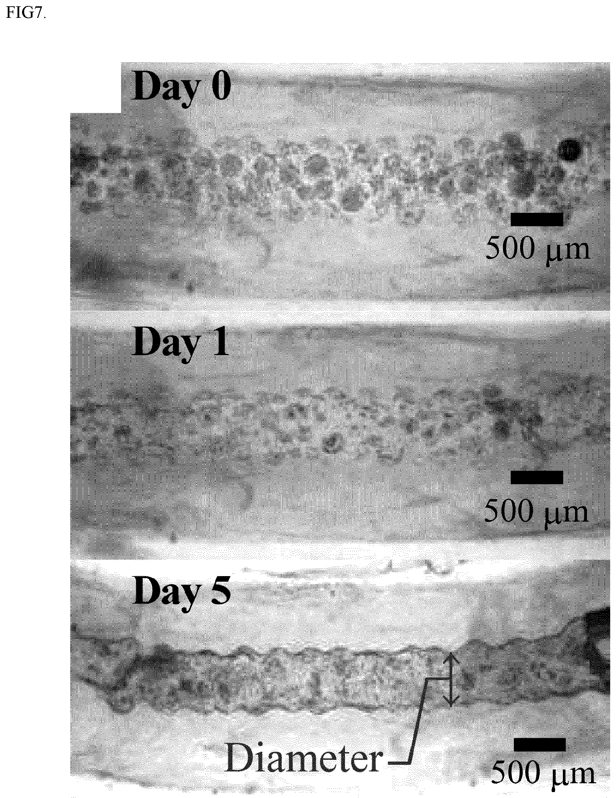

Fig. 7 is a photograph as a view showing the Caco-2 cell in the artificial intestine.

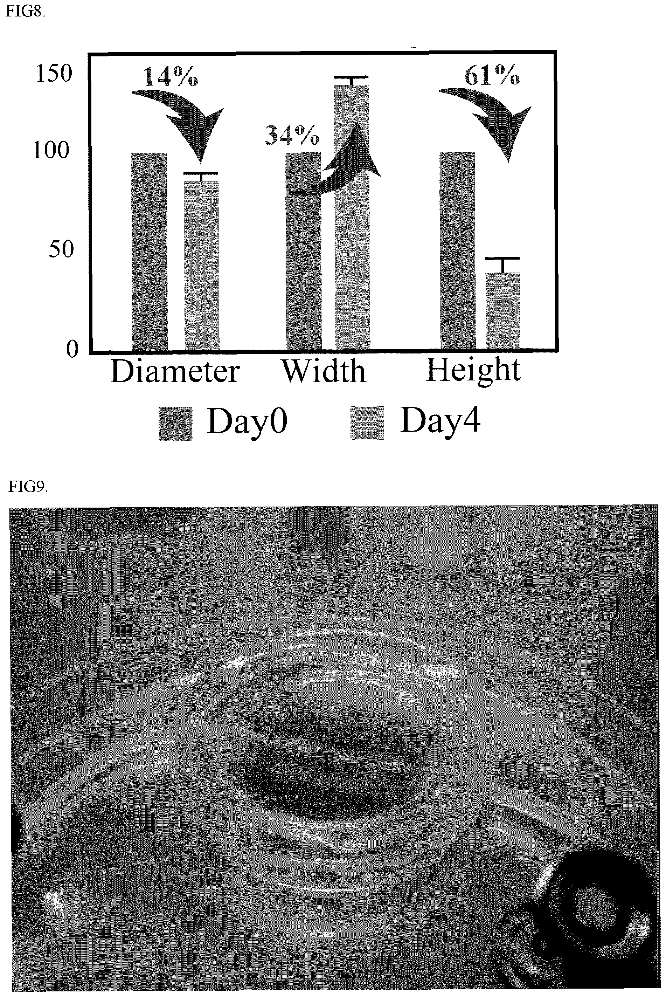

Fig. 8 is a graph as a view showing the diameter of the tube of the artificial intestine, the depth of the recess formed in the lumen, and the diameter of the opening of the recess. As shown in Fig. 8, after cultivation for four days, the diameter of the tube decreased by 14%, the depth of the recess decreased by 61%, and the diameter of the opening of the recess increased by 34% due to the traction force of the cell.

### [Experimental Example 3]

### Cocultivation of Human Cell and Intestinal Bacterium

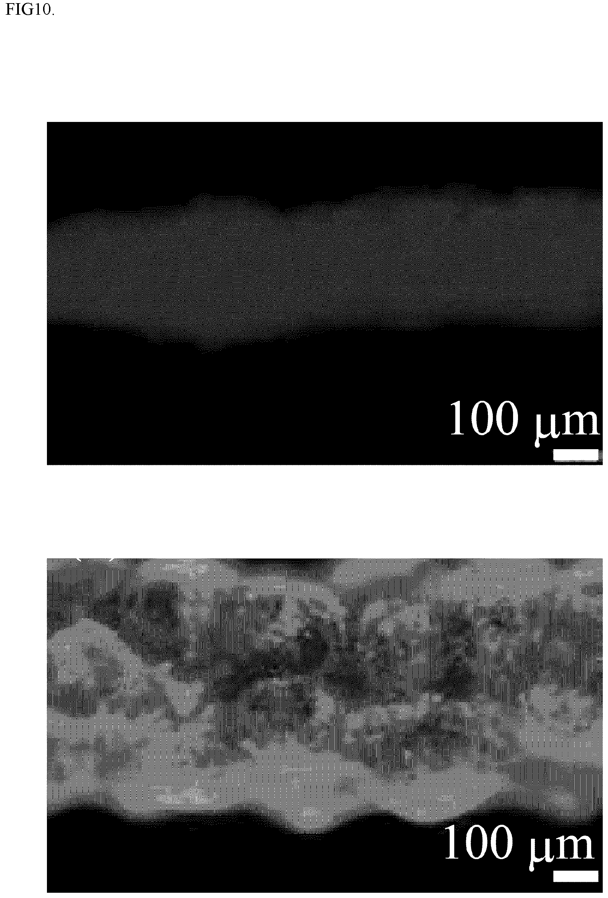

Next, cocultivation was performed on the artificial intestine using bifidobacteria. Fig. 9 is a photograph as a view showing a cocultivation system. The bifidobacteria (5.0 × 10⁸ cells/mL) were spread into the resultant artificial intestine, and the resultant was left stand for 24 hours for cultivation. The fluorescently stained bifidobacteria were observed. In this example, the artificial intestine was placed in a medium in which the human cell (Caco-2 cell) is cultivated, and a medium in which the intestinal bacteria (bifidobacteria) are cultivated flowed in and out of the openings of both end portions of the artificial intestine. Fig. 10 is a fluorescence photograph as a view showing the results of cocultivation. The upper and lower views show the photographs of the intestinal bacteria (bifidobacteria) and the human cell (Caco-2 cell), respectively. As shown in Fig. 10, the results show that the bifidobacteria stayed in the lumen of the artificial intestine.

### [Experimental Example 4]

### F-hiSIEC Cultivation Procedure

### Cultivation Method

Cell seeding was performed in such a manner that F-hiSIECTM (FUJIFILM human iPS cell-derived Small Intestinal Epithelial like Cell) is suspended at a concentration of 4.0 × 10⁶ cells/mL in a dedicated seeding medium and is separately delivered multiple times to the formed tube.

On the day after cell seeding, the medium was switched to a dedicated cultivation medium, and thereafter, was replaced every three days. Upon medium replacement, the solution delivery to the lumen of the tube was also performed, and in this manner, a dead cell was removed simultaneously.

Cultivation in the tube was performed for nine days.

### Observation Results

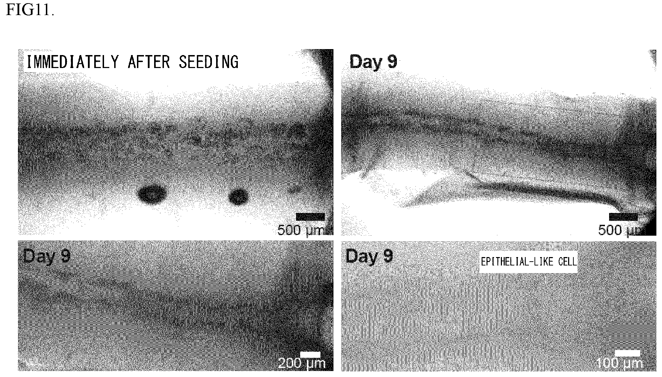

Fig. 11 shows an image captured by a phase-contrast microscope. Fig. 11 shows, on the upper left side, a state immediately after seeding. Fig. 11 shows, on the upper right side, a state after a lapse of nine days. Fig. 11 shows, on the lower left side, a partially enlarged view of the upper right view of Fig. 11. Fig. 11 shows, on the lower right side, an enlarged view of the lower left view of Fig. 11. As a result of observation with the phase-contrast microscope, growth of the cell and formation of an intestinal epithelial-like tissue in the lumen were confirmed. Further, a phenomenon was observed, in which a tube inner wall was pulled in a contraction direction due to action of the traction force of the cell accompanied by formation of the intestinal epithelial-like tissue and the tube inner diameter decreases accordingly.

The key point of the present invention is that many 100 µm order asperities observed in an inner wall of an intestine of a biological body can be three-dimensionally and instantaneously produced. Further, only a general arbitrary waveform generator is required for production, and a special device is not necessary. Thus, a barrier to introduce this technique is reduced.

The material of the tube is not limited to the gel. The tube can be produced if two conditions including a condition where the recesses can be produced by the bubbles and a condition where the recesses can be maintained even after removal of the bubbles are satisfied.

The method for generating the air bubbles is not limited to electrolyzation. Examples of this method include a method using a tubular rod-shaped mold capable of delivering air and provided with an air outlet in the surface thereof, a method of generating dissolved gas by heating a rod-shaped mold, and the like.

### INDUSTRIAL APPLICABILITY

This invention relates to an artificial intestine and the like, and therefore, is useful in the field of medical devices, the field of biological experimental devices, and the like.

## Claims

1. A method for manufacturing a luminal structure, comprising:
an in-cast temporary fixative obtaining step of obtaining an in-cast temporary fixative as a temporary fixative housed in a cast having a recess corresponding to an outer shape of the luminal structure, a core forming member corresponding to a lumen body of the luminal structure being inserted in the temporary fixative; and
an in-cast temporary fixative solidifying step of solidifying the in-cast temporary fixative in a state of a bubble being generated in the in-cast temporary fixative using the core forming member.

2. The method for manufacturing the luminal structure according to claim 1, wherein
the luminal structure is an artificial intestine.

3. The method for manufacturing the luminal structure according to claim 1, wherein
the temporary fixative is a pregel solution.

4. The method for manufacturing the luminal structure according to claim 3, wherein
the core forming member is an electrode, and
the in-cast temporary fixative solidifying step includes an electrolyzation step of generating the bubble on a surface of the core forming member by electrolyzation using the core forming member as the electrode.

5. The method for manufacturing the luminal structure according to claim 4, wherein
the electrolyzation step adjusts a value of voltage applied to the core forming member and a time of voltage application to adjust a shape of asperities inside the resultant luminal structure.

6. A luminal structure having a tubular shape, comprising
a lumen, wherein
the lumen includes a plurality of recesses having a depth of no less than 10um and no more than 200µm, and a maximum width of no less than 20um and no more than 500um..

7. The luminal structure according to claim 6, wherein
the luminal structure has 10² or more of the recesses.
